# EUROPEAN PATENT SPECIFICATION

(11) **EP 2 116 210 B1**
(45) Date of publication and mention of the grant of the patent: **25.04.2012**
(21) Application number: 08155701.9
(22) Date of filing: 06.05.2008
(51) Int. Cl.: A61F 2/00

(54) **Knee implant**
Knieimplantat
Implant du genou

(43) Date of publication of application: 11.11.2009
(73) Proprietor: Episurf Medical AB, 115 42 Stockholm (SE)
(72) Inventor: Larsson, Stig, 147 63 Uttran (SE); Ryd, Leif, 113 40 Stockholm (SE); Esmaeilzadeh, Saeid, 113 54 Stockholm (SE); Flodström, Katarina, 113 62 Stockholm (SE); Wang, Gang, 187 36 Täby (SE)
(74) Representative: Kitzler, Michael

(56) References cited:
- EP-A- 0 545 718
- WO-A-2007/121242
- US-A1- 2007 116 734
- US-B1- 6 306 925

## Description

### Field of the invention

The present invention relates to a medical implant for replacing cartilage at an articular surface. The new implant component can be inserted into the body through a small surgical operation like arthroscopy and further, it is possible to tailor the component for each individual patient.

### Background

The knee provides support and mobility and is the largest and strongest joint in the body. Pain in the knee can be caused by for example injury, arthritis or infection. The friction between the cartilage and the joint is very low, which facilitates the movement of the joint under high pressures. Cartilage does not heal after damages, as opposed to other connective tissue. This means that damages of the cartilage gradually become worse. Another part of the problem with damaged knee joints is that the joints are very sensitive for pain.

Today's knee prostheses are successful in relieving pain but there is a limit in the lifetime of the prostheses of 10-15 years. The operation is demanding and the convalescence time is often around 6 months. In many cases today, surgery is avoided if training and painkillers can reduce the pain. Prostheses are therefore foremost for elderly patients in great pain. There are different kinds of prostheses, such as half prosthesis, total prosthesis and revision knee, the latter used after a prosthesis failure. In general the knee prostheses are made up of two separate components, one on the tibial side and one on the femoral side. The materials used in today's knee prostheses are often a combination of a metal and a polymeric material, but other materials such as ceramics have also been used. The knee prostheses are inserted through open surgery. There is a lack of small wear resistant knee implants which can be inserted through arthroscopy or a small open surgical operation, in order to replace damaged cartilage. US2003060887, WO2004075777, WO2005084216, WO2006004885, WO2006091686, US2007179608 describe implants in this field. However, even smaller versions are desirable, where the influence on the surrounding parts of the knee is as small as possible. Further, a small implant comprising a bioactive material that biochemically instead of mechanically attaches to the bone is even more desirable.

A functionally graded material (FGM) is characterised by a gradual change of material properties with position. It is an anisotropic composite material where a gradient deliberately has been introduced into the material. This gradient can for example be in composition and/or microstructure. FGMs can be prepared through different techniques such as conventional powder metallurgy processing, vapour deposition and sintering techniques.

Electric pulse assisted consolidation (EPAC) includes processes based on heating a material to be compacted with a pulsed DC current. Other names commonly used for this technique are spark plasma sintering (SPS), pulsed electric current sintering (PECS), field assisted sintering technique (FAST), plasma-assisted sintering (PAS) and plasma pressure compaction (P²C). The process allows very rapid heating under high pressures. This process, hereafter referred to as SPS, has proved to be very well suited for the production of functionally graded materials. SPS gives advantages such as no need of binders in the powders and a controlled shrinkage of the material during the compaction. Further, the possibility to rapidly change the temperature and pressure makes it easier to tailor the microstructure of the material and to optimize the sintering conditions compared to conventional compaction techniques. WO2006/083603 describes the production of a FGM net shaped body with FAST/SPS where the different materials included are a metal or a metal alloy in combination with a ceramic such as an oxide, nitride or carbide, or another metal or metal alloy.

FGMs have previously been suggested as suitable for medical implants, mainly for dental applications. Examples of such materials are zirconium dioxide/hydroxylapatite as in CN1709829 and titanium/apatite as in WO2007069623. Gradient materials formed from biodegradable polyesters combined with hydroxylapatite and formed as tailor-made implant components, individual for each patient, have been suggested for cranial reconstruction (Pompe et al., Materials Science and Engineering A, 362, 2003, 40-60). Other combinations of materials for implant purposes are metals coated with a bioactive coating such as hydroxylapatite. There is however still a need for improved implant materials with a combination of excellent adhesion to the body and desired mechanical properties.
Document WO 2007/121242 A2 discloses a medical implant for an articulate surface made of a ceramic metal composite. The composite includes one phase that is a biocompatible metal or metal alloy and a second phase of ceramic particles of carbides, nitrides borides and/or oxides. The purpose of the ceramic in the composite is to provide hardening of the metal/metal alloy and reducing metal-ion release from the articulate surface. The implant comprises an articulating surface and a non articulating substrate. The articulate surface comprises at least 1-100% ceramic material. Beneath the articulate surface there is a graded layer ceramic/metal composite having decreasing amounts of ceramic and increasing amounts of metal, either to pure metal or to a steady state ceramic/metal composition within the substrate. The graded structure may be formed as step wise layers or be linear. The graded structure may be achieved by powder metallurgical processes such as sintering.
US 6306925 B1 discloses a functionally graded bioactive composite material in the shape of a casted tape. The composite material comprises an outer layer of bioactive glass, a layer of biocompatible ductile metal or metal alloy (e.g. stainless steel, titanium, aluminum or cobalt-chrome alloy) and one or more interlayers. The interlayers are designed to produce an interface between the metal and the bioactive layer and should be intermediate to the metal and bioactive layers in tortuosity. The interlayers may be of metal or ceramic powders, e.g. layers of ductile metals or of alumina, hydroxyapatite, zirconia etc. Different combinations of interlayers are conceivable, e.g. two layers of ductile metal, optionally with an additional a layer of a ceramic material such as alumina or hydroxyapatite. The composite material is manufactured using a tape casting process, wherein powders of bioactive glass, metal or ceramic respectively are mixed with organic binder, plasticizers and dispersants to slurry which is then formed into a tape. Tapes are then combined into laminates and organic compounds removed before sintering the laminate into a multilayered tape.

### Brief description of the invention

The present invention is defined in claim 1 and concerns a medical implant for replacing cartilage at an articular surface. The implant has a design that can be tailored to replace the damaged cartilage for each individual knee. The material is a functionally graded material consisting of a bioactive material such as hydroxylapatite and a hard, wear-resistant biocompatible material such as stainless steel, where the bioactive material easily attaches to the bone while the hard material gives the desired mechanical properties for the load-bearing surface. The implants made of the functionally graded material have superior mechanical stability compared to previously known implants.

### Brief description of the figures

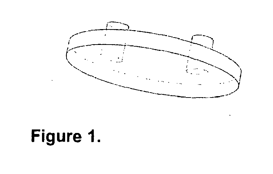

Figure 1. A schematic drawing of the 3D view of one embodiment of the knee implant component.

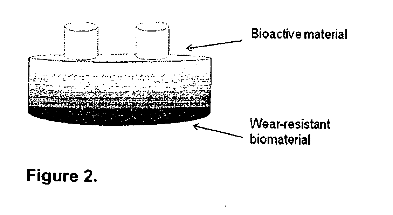

Figure 2. A schematic drawing of one embodiment of the knee implant component, with one side being a bioactive material, the other side being a wear resistant biomaterial and the two sides having a compositional gradient inbetween.

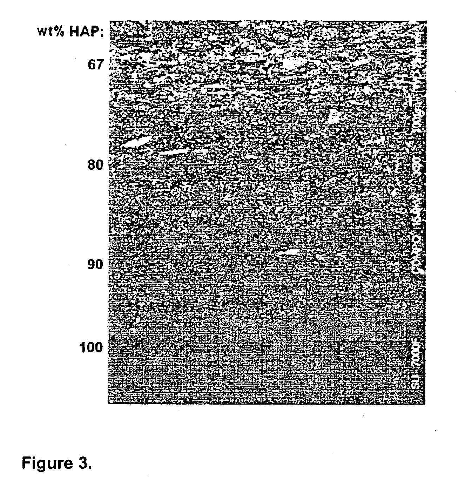

Figure 3. A scanning electron micrograph showing four different layers of a FGM according to the present invention. The four layers contain 100 wt%, 90 wt%, 80 wt% and 67 wt% hydroxylapatite respectively and the rest is stainless steel 316L.

### Detailed description of the invention

The present invention relates to an articular surface implant such as a knee implant. Initially, the implant is intended for the knee joint, but principally other joints such as elbow, ankle or finger joints can be candidates. The knee joint is the joint most often suffering from the conditions approachable by this implant.

A method for producing a functionally graded material with a biocompatible wear resistant material having a gradual transition to a bioactive part, said biocompatible wear resistant material being a metal or metal alloy, comprises the steps:
i) forming a gradient material composed of at least 4 layers, wherein one layer essentially comprises 100 wt% biocompatible wear resistant metal or metal alloy, and the other layers each essentially comprise a powder of a biocompatible wear resistant metal or metal alloy, together with a powder of a bioactive material, or the bioactive material only, wherein each of the layers differ in the ratio between wear resistant metal or metal alloy and bioactive material;
ii) consolidating the gradient material of step i) by using a sintering technique; wherein the layer with essentially 100 wt% metal or metal alloy has a relative density of at least 95 % of the theoretical density.

The sintering technique is preferably electric pulse assisted consolidation, also referred to as spark plasma sintering (SPS).

It is preferred that said relative density is at least 97% or at least 99%.

It is preferred that the ratio between said wear resistant metal or metal alloy and said bioactive material in any of the layers is in the range of 100:0 to about 0:100, or in the range of 100:0 to about 30:70, or in the range of 100:0 to about 50:50.

It is preferred that the number of layers is between 4 and 25, or between 7 and 20.

It is preferred that the wear resistant metal or metal alloy is stainless steel.

It is preferred that said wear resistant metal or metal alloy is stainless steel, preferably comprising iron, carbon, chromium (12-20%), molybdenum (0.2-3%), and nickel (8-15%).

It is preferred that the sintering temperature of the SPS process is between 800°C and 1300°C, preferably between about 900°C and about 1100°C.

It is preferred that the sintering time of the SPS process is between 1 and 30 minutes, typically between 2 and 10 minutes.

It is preferred that said bioactive material is selected from the group comprising; hydroxylapatite, calcium sulphate, calcium phosphates, calcium aluminates, calcium silicates or calcium carbonates, or mixtures thereof.

It is preferred that the bioactive material further comprises one or more additives chosen from the group comprising oxides of phosphorous, sodium, magnesium, potassium, silver, aluminium, titanium and/or silicon.

It is preferred that the number of layers in the functionally graded material is between 4 and 25, or preferably between 5 and 15, wherein said bioactive material, or the layers comprising said bioactive material, further comprises a maximum of about 50 wt% stainless steel, or a maximum of about 30% stainless steel.

It is preferred that one of the layers essentially comprises stainless steel and one of the layers essentially comprises the bioactive material, and other layers, if present, essentially comprise a mixture of stainless steel and the bioactive material.

It is preferred that a component comprising the material according to the above has an outer surface area of the wear-resistant biomaterial layer between about 0.5 cm² and about 20 cm², or preferably between about 1 cm² and about 10 cm², and/or having a thickness between about 1 mm and about 10 mm, preferably between about 2 mm and 5 mm.

To facilitate the attachment of the component above to the bone, the component has at least 1, preferably 2 to 3, pegs, comprising a bioactive material, said pegs protruding from the bioactive surface layer to facilitate adhesion of the implant.

The bioactive material of the pegs is selected from the group comprising; hydroxylapatite, calcium sulphate, calcium phosphates, calcium aluminates, calcium silicates or calcium carbonates, or mixtures thereof.

The bioactive material of the pegs may comprise an additive of maximum 10 wt% of oxides of materials from the group of phosphorous, sodium, magnesium, potassium, silver, aluminium, titanium and/or silicon.

The bioactive material of the pegs may further comprise a maximum of about 50 wt% stainless steel, or a maximum of about 30 wt% stainless steel.

A component according to the above may be used as an implant. The implant may be tailored for each individual person where it is to be implanted. The implant may be produced after drawings obtained after a CT scan or MRI of the specific part of the body intended for the implant.

The invention provides an implant composed of the functionally graded material made according to the above, and according to the examples herein.

The invention provides an implant according to the above, where the tailored shape is obtained through production in a tailor made mould, or through post sintering treatment, including cutting, grinding and polishing.

The implant may be used in a knee, for example to replace damaged cartilage.

The implant may be inserted into the human body through surgical operation, such as through arthroscopy.

The implant of the present invention may be used on both sides in the knee, that is as two separate components, one on the tibial side and one on the femoral side.

The implant component of the present invention comprises at least two different materials and is formed as a gradient material having a wear-resistant load-bearing surface on one side and having another side with a bioactive material that easily attaches to the bone.

The wear-resistant biocompatible material can consist of a metal, a metal alloy such as stainless steel or a ceramic material. More specifically it can consist of any metal or metal alloy used for structural applications in the body, such as stainless steel, cobalt-based alloys, chrome-based alloys, titanium-based alloys, pure titanium, zirconium-based alloys, tantalum, niobium and precious metals and their alloys. If a ceramic is used as the biocompatible material, it can be a biocompatible ceramic such as aluminium oxide, silicon nitride or yttria-stabilized zirconia. For stainless steel, different grades exist where the properties have been optimised for applications in the human body. The preferred stainless steel for use in the present invention is an alloy mainly comprising iron, carbon, chromium (12-20%), molybdenum (0.2-3%), and nickel (8-15%). Stainless steel is a material which is well documented for the application in implants and prostheses. Further, the mechanical properties of stainless steel are for some purposes superior to those of ceramic materials, due to the brittleness of the ceramics.

The bioactive part of the FGM of the present invention can typically be hydroxylapatite. Hydroxylapatite (also called hydroxyapatite) has the chemical composition Ca₅(PO₄)₃(OH), but is usually written Ca₁₀(PO₄)₆(OH)₂ to denote that the crystal unit cell comprises two molecules. More specifically, the bioactive part of the FGM is chosen from the group consisting of hydroxylapatite, calcium sulphate, calcium phosphate, tricalcium phosphate, calcium aluminate, calcium silicate or calcium carbonate, or mixtures of these materials. Further, small amounts, up to 10 wt%, of sintering additives such as oxides of phosphorous, sodium, magnesium, potassium, silver, aluminium, titanium and/or silicon can be added to the hydroxylapatite powder to facilitate the sintering process. The outermost layer of the bioactive side of the implant may further include up to approximately 50 wt% stainless steel for improved mechanical properties, however, there is a balance between improved mechanical properties and reduced bioactivity.

In addition, the material may comprise three different materials (e.g. steel, titanium, and hydroxylapatite) to obtain improved compatibility between the layers and thereby reduce the number of layers and avoid undesired cracks in the layers. Such a FGM could have for example titanium, a titanium alloy or a ceramic material as the third material between the wear-resistant biocompatible material and the bioactive material.

For FGMs produced through sintering, a small particle size of all the raw materials, such as <20 µm, is desirable for a uniform sintering result. Further, a high purity powder is important for implant applications. For the spark plasma sintering, the powders are mixed and dried according to conventional techniques.

The size of a FGM component produced through spark plasma sintering can vary depending on the application of the component as well as on limitations of the SPS equipment. For the implant purposes of the present invention however, the small size of the particular component makes it possible to produce it in a small version of a SPS unit. and The size of the component can be varied and adjusted to the need for the particular patient. The cross section of the component should be roughly circular or slightly elongated, with a surface area of between about 0.5 cm² and about 20 cm², or typically between 1 cm² and 10 cm². The implant can also be of a similar size but with a less defined shape. The thickness of the implant is in the region of about 1 mm to about 10 mm, typically from about 2 mm to about 5 mm. The implant is typically attached to one side of the knee joint to replace the damaged cartilage, and the thickness of the implant should match the thickness of the original cartilage layer.

The gradient material of the present invention consists of a number of layers, typically between 4 and 25, or more typically between 7 and 20. , with different compositions in each layer. The outermost layers of the component consist of the biocompatible material, forming a load-bearing surface, and the bioactive material, with or without addition of the wear-resistant material for improved stability, forming a bone-contacting surface, respectively. The surface of the bioactive material can be rough in order to facilitate the attachment of the implant to the bone. The surface of the hard biomaterial should typically be convex, based on the original surface contour. The surface of the biomaterial should typically be flat or concave to fit the underlying bony surface, with the exception for some protrusion, at least 1 and typically 2 to 3 pegs, for attaching the implant into the skeleton. These pegs should comprise the bioactive material and have a length of 1 to 10 mm, typically 2-5 mm, and a diameter of 1 to 5 mm, typically 2 to 4 mm. When in place in the joint, the pegs should penetrate the sub-chondral boneplate and engage the cancellous bone. The protrusion will give stability and promote the attachment of the bioactive material to the bone. Typically, the implant will be custom made for a particular joint of a particular patient in order for the curvature of the implant component to be identical to that of the sub-chondral bone plate at the site of insertion. An alternative to custom made implants is to create a kit of implant components of different sizes and shapes available, implying no need for individual tailoring.

The general way to form a FGM through spark plasma sintering today is to build it up layer by layer with different compositions. However, there is nothing that limits this invention to a functionally graded material with well defined layers. A different filling technique for addition of the different powder compositions could give less defined layers but could still lead to desired properties of the FGM. Further, prior to sintering, the powders can be pressed together and thereby form a so called green body, which is an un-sintered ceramic item, which is later on inserted into the SPS unit to be sintered.

The spark plasma sintering technique is used for manufacturing the gradient components of this specific invention. However, gradient materials produced through alternative methods can also be used for the same purpose, for example sintering methods such as hot pressing, hot isostatic pressing or pressureless sintering. SPS combines rapid heating, a short holding time at the desired sintering temperature and a high pressure for sintering of the components.

The pressure used during the process is between 10 and 150 MPa, preferably between 30 and 100 MPa. The heating rate applied is between 5 and 600 °C min⁻¹, preferably 50 -150 °C min⁻¹. The sintering temperature and time are chosen so that a total or near total densification, implying a density of at least 95%, or at least 97%, or at least 99% of the theoretical density, will be obtained for the layer comprising essentially 100 wt% biocompatible wear resistant material. This temperature is between 800°C and 1300°C, typically 900°C -1100°C. The holding time during the sintering process is between about 1 minute and about 30 minutes, typically between about 2 minutes and about 10 minutes.

The SPS technique uses a combination of a high current and a low voltage. A pulsed DC current with typical pulse durations of a few ms and currents of 0.5-30 kA flows through the punches, die and, depending on the electrical properties of the specimen, also through the specimen. The electrical pulses are generated in the form of pulse packages where the on: off relation is in the region of 1:99 to 99:1, typically 12:2 (12 pulses on, 2 off). The pressure is applied on the two electrically conducting punches of the powder chamber, in a uniaxial direction.

A method may comprise the following steps: (i) performing a CT scan or MRI on the patient in order to determine the desired size and shape of the implant component; (ii) creating graphite tools for the spark plasma sintering unit shaped according to CAD-drawings created according to results from the CT scan/MRI; (iii) inserting the powders of different compositions stepwise, or a pre-formed green body, into a conductive graphite die (chamber); (iv) closing the graphite die chamber with two electrically conducting punches and inserting the die into the SPS unit; (v) applying a uniaxial pressure on the two punches of the graphite die, thereby applying a pressure on the material; (vi) heating the material with a pulsed electrical energy, going through the punches and the electrically conducting die, during a desired time until the sintering is completed.

The sintering procedure includes a high heating rate (a steep heating ramp) and a short holding time at the desired sintering temperature. The cooling down of the sample can either be programmed or the sample will cool down automatically as the current is switched off.

In another method the component is formed according to a standard size and shape, typically a cylindrical or slightly elongated component, and that the individual design is obtained through post compaction grinding of the gradient material according to drawings created for the specific patient.

A kit may be provided which contains a selection of implants according to the invention, wherein the selection of implants comprises implants of different sizes and shapes. This means that an implant according to the invention, which is of suitable size and shape for the patient to be treated, is available for the surgeon who adjusts the excision of the cartilage to the available components.

The implant is introduced into the joint by surgical treatment, such as by means of a closed procedure, i.e. by arthroscopy as opposed to open surgery, or a small open surgical operation which is much smaller than operations for today's knee prostheses. This provides for minimal postoperative morbidity. The implant is not conceived of as a joint prosthesis but rather as an artificial biomaterial for installation into a portion of an articular surface, i.e. for cartilage replacement. Prosthetic replacement may become necessary at a later stage and the present procedure should preferably not interfere with subsequent joint arthroplasty. It is therefore important with an implant, according to the present innovation, that is not interfering with a possible future prosthesis. The use of biomaterials in this procedure implies both a wear-resistant component as well as a bioactive component, as opposed to biological solutions using cell or tissue transplants.

Initially, the knee joint will be addressed, but principally other joints such as elbow, ankle or finger joints can be candidates. The knee joint is the joint most often suffering from the conditions approachable by this implant.
For the surgical treatment, either of two situations may be at hand: 1) a chondral fracture, where the sub-chondral bone plate typically is completely denuded of cartilage over a confined area with sharp edges to the surrounding intact cartilage or 2) a focal area of degenerated cartilage.

Two surgical methods may comprise the following steps:
1. With a chondral fracture, the implant is manufactured to be a perfect fit to the cartilaginous defect as outlined by preoperative CT/MRI investigations. Ordinary arthroscopy is used with the patient in either general anesthesia, spinal block or even local anestesia. Standard instruments are used, available at any orthopaedic institution. Whether to use saline or gas arthroscopy is determined empirically. Both options are available. The area of interest on the femur is brought into vision by properly flexing the joint. Holes, corresponding to the fixation pegs on the implant, are drilled into the sub-chondral bone. Further, the remaining bone surface will be machined as to draw blood in order for adherence to the bioactive layer of the implant to occur. The implant is inserted into the joint and the fixation pegs are made to engage into the corresponding holes. Subsequently, the implant is hammered in place. The thickness of the implant is such that it matches that of the surrounding cartilage. The edges are slightly undercut so as to become somewhat countersunk below the level of the surrounding cartilage. Initial fixation of the implant occurs by interference fit between fixation pegs and the corresponding holes. Definite fixation is obtained by the bioactive layer bonding with host bone.
2. With focal areas of degeneration, the transition to normal cartilage is gradual and not suitable for an implant as above. This will require a two-stage procedure. Here it is anticipated that a primary arthroscopy is made and some cartilage is burred away around the edges of the damaged area in order to create a situation as above. Subsequently, CT/MRI is performed and the procedure is then carried out as above.

For some joints, it is anticipated that the two stages in point 2 above can be made at one operative procedure where a cartilage defect is "created" through excision to fit an implant that is prefabricated to fit the underlying bone. In this case the operation will be performed as an open, as opposed to scopic, procedure.

### Examples

### Example 1

A gradient material of hydroxylapatite (HAP) and stainless steel (SS) was prepared. 11 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |
| 7 | 40 | 60 |
| 8 | 30 | 70 |
| 9 | 20 | 80 |
| 10 | 10 | 90 |
| 11 | 0 | 100 |

The 11 different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die chamber and the chamber was closed by two punches. The sample was sintered in a SPS unit and the temperature was initially automatically raised to 600°C. Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 100 MPa. The component was shaped as a cylinder with a diameter of 20 mm and a height of 5 mm. The layers were free of cracks.

### Example 2

A gradient material of hydroxylapatite (HAP) and stainless steel (SS) was prepared. 8 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |
| 7 | 40 | 60 |
| 8 | 30 | 70 |

The eight different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die chamber and the chamber was closed by two punches. The sample was sintered in a SPS unit and the temperature was initially automatically raised to 600°C. Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 75 MPa. The component was shaped as a cylinder with a diameter of 20 mm and a height of 4 mm. The layers were free of cracks.

### Example 3

A gradient material of hydroxylapatite (HAP) and stainless steel (SS) was prepared. 6 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |

The six different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die and the die was closed by two punches. The sample was sintered in a SPS unit and the temperature was initially automatically raised to 600°C.
Subsequently, a heating rate of 100°C min⁻¹ was applied. The sample was densified at 950°C for 5 minutes. The temperature was measured with an optical pyrometer focused on the surfaces of the sintering die. The sintering took place under vacuum. The pressure was kept at 100 MPa. The component was shaped as a cylinder with a diameter of 20 mm and a height of 3 mm. The layers were free of cracks.

### Example 4

A knee with damaged cartilage was scanned with CT and the result thereof was converted to a CAD-drawing. Graphite tools for the SPS chamber were formed according to the requirements from the CAD-drawing in order to sinter a tailor made component in the graphite tool. The lower punch had two holes for formation of the bioactive pegs. A stainless steel/hydroxylapatite gradient material was formed through spark plasma sintering according to Example 1, with 11 different layers. The sample was densified at 1000 °C for 5 minutes. The sintering took place under vacuum and the pressure was 75 MPa.

### Example 5 (comparative example)

A gradient material of hydroxylapatite (HAP) and stainless steel (SS) was prepared. 6 different powder mixtures were prepared with the following compositions:

| Layer | wt% SS | wt% HAP |
|---|---|---|
| 1 | 100 | 0 |
| 2 | 80 | 20 |
| 3 | 60 | 40 |
| 4 | 40 | 60 |
| 5 | 20 | 80 |
| 6 | 0 | 100 |

The six different powders were produced through mixing stainless steel 316L (D₉₀<22 µm) and/or hydroxylapatite (D₅₀<5 µm) in a liquid medium in a ball mill for 1 h followed by drying in a conventional oven. The powders were inserted layer by layer in a graphite die and the die was closed by two punches. The sample was sintered in a SPS unit and the temperature was initially automatically raised to 600°C.
Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 75 MPa. The component was shaped as a cylinder with a diameter of 20 mm and a height of 3 mm. The pure hydroxylapatite layer at one side of the cylinder cracked and the experiment was therefore not successful. Compared to the material according to this example, the functionally graded material according to the invention is more mechanically stable.

## Claims

1. A medical implant for replacing cartilage at an articular surface comprising a functionally graded material with at least 4 layers, wherein
a first layer comprises a load bearing surface consisting of a biocompatible wear resistant material, said layer and surface comprising essentially 100 wt% biocompatible metal or metal alloy ; and
a second layer having a bone-contact surface comprises a bioactive material ; said load bearing surface and bone contact surface facing mutually opposite directions ;
the remaining layers forming a compositional gradient, with a gradual transition in material composition from the first layer to the second layer , wherein each layer comprises the biocompatible wear resistant material and the bioactive material, and wherein the ratio of biocompatible wear resistant material to bioactive material gradually decreases going from the first layer towards the second layer ;
said functionally graded material being produced by a sintering technique, wherein each layer is formed from a powder of the biocompatible wear resistant material and/or the bioactive material having said ratios and the gradient material being consolidated by using a sintering technique so that the first layer, with essentially 100 wt% biocompatible metal or metal alloy, has a relative density of at least about 95% of the theoretical density, wherein
a peg comprising the bioactive material protrudes from the bone-contact surface.

2. The medical implant of claim 1, wherein the surface area of the implant is between about 1 cm² and 10 cm² and the thickness of the implant is about 2 mm to about 5mm.

3. The medical implant of claims 1-2, wherein the biocompatible wear resistant material is any of stainless steel, cobalt-based alloys, chrome-based alloys, titanium-based alloys, pure titanium, zirconium-based alloys, tantalum, niobium and precious metals and their alloys.

4. The medical implant of any of the preceding claims, wherein the bioactive material is any of hydroxylapatite, calcium sulphate, calcium phosphate, tricalcium phosphate, calcium aluminate, calcium silicate or calcium carbonate, or mixtures of these materials.

5. The medical implant of any of the preceding claims, wherein the biocompatible wear resistant material is stainless steel.

6. The medical implant of any of the preceding claims, wherein the bioactive material is hydroxylapatite.

7. The medical implant of any of the preceding claims, wherein the sintering technique is electric pulse assisted consolidation, also referred to as spark plasma sintering.

8. The medical implant of any of the preceding claims, wherein said relative density is at least about 97% or at least about 99%.

9. The medical implant of any of the preceding claims, wherein the ratio between said wear resistant metal or metal alloy and said bioactive material in any of the layers is in the range of 100:0 to about 0:100.

10. The medical implant of any of the preceding claims, wherein the ratio between said wear resistant metal or metal alloy and said bioactive material in any of the layers is in the range of 100:0 to about 30:70.

11. The medical implant of any of the preceding claims, wherein the ratio between said wear resistant metal or metal alloy and said bioactive material in any of the layers is in the range of 100:0 to about 50:50.

12. The medical implant of any of the preceding claims, wherein the number of layers is between 4 and 25, or between 7 and 20.

## Patentansprüche

1. Ein medizinisches Implantat zum Ersetzen von Knorpel an einer Gelenkoberfläche, das ein funktional abgestuftes Material mit mindestens 4 Schichten umfasst, wobei
- eine erste Schicht eine tragfähige Oberfläche umfasst, die aus einem biokompatiblen verschleißbeständigen Material besteht, wobei die Schicht und die Oberfläche im wesentlichen 100 Gew.% biokompatibles Metall oder eine Metall-Legierung umfassen; und
- eine zweite Schicht mit einer Knochenkontakt-Oberfläche, die ein bioaktives Material umfasst;
- wobei die tragfähige Oberfläche und die Knochenkontakt-Oberfläche in wechselseitig entgegengesetzte Richtungen weisen;
- wobei die übrigen Schichten einen Zusammensetzungsgradienten bilden, mit einem allmählichen Übergang in der Materialzusammensetzung von der ersten Schicht zu der zweiten Schicht, wobei jede Schicht das biokompatible verschleißbeständige Material und das bioaktive Material umfasst, und wobei das Verhältnis von biokompatiblem verschleißbeständigen Material zu bioaktivem Material sich von der ersten Schicht in Richtung auf die zweite Schicht allmählich verringert;
- wobei das funktional abgestufte Material durch eine Sintertechnik hergestellt wird, wobei jede Schicht aus einem Pulver des biokompatiblen verschleißbeständigen Materials und/oder des bioaktiven Materials mit den Verhältnissen gebildet wird, und das Gradientenmaterial durch Verwenden einer Sintertechnik verfestigt wird, so dass die erste Schicht, mit im Wesentlichen 100 Gew.% biokompatiblen Metalls oder einer Metall-Legierung eine relative Dichte von mindestens etwa 95% der theoretischen Dichte aufweist;
- wobei ein Stab, der das bioaktive Material umfasst, von der Knochenkontakt-Oberfläche vorragt.

2. Das medizinische Implantat nach Anspruch 1, wobei die Fläche der Oberfläche des Implantats zwischen etwa 1 cm² und 10 cm² und die Dicke des Implantats zwischen 2 mm bis etwa 5 mm beträgt.

3. Das medizinische Implantat nach Anspruch 1 oder 2, wobei das biokompatible verschleißbeständige Material eines ist aus Edelstahl, auf Kobalt basierenden Legierungen, auf Chrom basierenden Legierungen, auf Titan basierenden Legierungen, purem Titan, auf Zirconium basierenden Legierungen, Tantal, Niob oder Edelmetallen oder deren Legierungen.

4. Das medizinische Implantat nach einem der vorhergehenden Ansprüche, wobei das bioaktive Material eines ist aus Hydroxylapatit, Kalziumsulfat, Kalziumphosphat, Trikalziumphosphat, Kalziumaluminat, Kalziumsilikat oder Kalziumkarbonat, oder Mischungen dieser Materialien.

5. Das medizinische Implantat nach einem der vorhergehenden Ansprüche, wobei das biokompatible verschleißbeständige Material Edelstahl ist.

6. Das medizinische Implantat nach einem der vorhergehenden Ansprüche, wobei das bioaktive Material Hydroxylapatit ist.

7. Das medizinische Implantat nach einem der vorhergehenden Ansprüche, wobei die Sintertechnik eine durch einen elektrischen Impuls unterstützte Verfestigung ist, die auch als Spark Plasma Sintern bezeichnet wird.

8. Das medizinische Implantat nach einem der vorhergehenden Ansprüche, wobei die relative Dichte mindestens etwa 97% oder mindestens etwa 99% beträgt.

9. Das medizinische Implantat nach einem der vorhergehenden Ansprüche, wobei das Verhältnis zwischen dem verschleißbeständigen Metall oder der Metall-Legierung und dem bioaktiven Material in einer der Schichten in dem Bereich 100:0 bis etwa 0:100 liegt.

10. Das medizinische Implantat nach einem der vorhergehenden Ansprüche, wobei das Verhältnis zwischen dem verschleißbeständigen Metall oder der Metall-Legierung und dem bioaktiven Material in einer der Schichten in dem Bereich 100:0 bis etwa 30:70 liegt.

11. Das medizinische Implantat nach einem der vorhergehenden Ansprüche, wobei das Verhältnis zwischen dem verschleißbeständigen Metall oder der Metall-Legierung und dem bioaktiven Material in einer der Schichten in dem Bereich 100:0 bis etwa 50:50 liegt.

12. Das medizinische Implantat nach einem der vorhergehenden Ansprüche, wobei die Anzahl der Schichten zwischen 4 und 25 oder zwischen 7 und 20 liegt.

## Revendications

1. Un implant médical pour remplacer du cartilage sur une surface articulaire comprenant un matériau fonctionnellement graduel avec au moins 4 couches, dans lequel
une première couche comprend une surface de support de charge composée d'un matériau biocompatible résistant à l'usure, ladite couche et ladite surface comprenant essentiellement 100% en poids de métal ou d'alliage métallique biocompatible, et
une deuxième couche ayant une surface de contact osseux comporte un matériau bioactif ;
ladite surface de support de charge et ladite surface de contact osseux étant tournées vers des directions mutuellement opposées ;
les autres couches formant un gradient de composition, avec une transition graduelle dans la composition du matériau depuis la première couche vers la deuxième couche, chaque couche comprenant le matériau biocompatible résistant à l'usure et le matériau bioactif, et le rapport du matériau biocompatible résistant à l'usure et du matériau bioactif, diminuant progressivement en allant de la première couche vers la deuxième couche ;
ledit matériau fonctionnellement graduel étant produit par une technique de frittage, dans laquelle chaque couche est formée à partir d'une poudre du matériau biocompatible résistant à l'usure et/ou du matériau bioactif ayant lesdits rapports, et le matériau graduel étant consolidé en utilisant une technique de frittage telle que la première couche, avec pratiquement 100% en poids de métal ou d'alliage métallique biocompatible, a une densité relative d'au moins environ 95% de la densité théorique,
dans lequel une cheville comprenant le matériau bioactif dépasse de la surface de contact osseux.

2. L'implant médical selon la revendication 1, dans lequel la surface de l'implant est comprise entre environ 1 cm² et 10 cm² et l'épaisseur de l'implant est d'environ 2 mm à environ 5 mm.

3. L'implant médical selon les revendications 1 et 2, dans lequel le matériau biocompatible résistant à l'usure est l'un des matériaux suivants : acier inoxydable, alliages à base de cobalt, alliages à base de chrome, alliages à base de titane, titane pur, alliages à base de zirconium, tantale, niobium et métaux précieux et leurs alliages.

4. L'implant médical de l'une des revendications précédentes, dans lequel le matériau bioactif est l'un des matériaux suivants : hydroxyapatite, sulfate de calcium, phosphate de calcium, phosphate tricalcique, aluminate de calcium, silicate de calcium ou carbonate de calcium, ou des mélanges de ces matériaux.

5. L'implant médical de l'une des revendications précédentes, dans lequel le matériau biocompatible résistant à l'usure est de l'acier inoxydable.

6. L'implant médical de l'une des revendications précédentes, dans lequel le matériau bioactif est de l'hydroxyapatite.

7. L'implant médical de l'une des revendications précédentes, dans lequel la technique de frittage est à consolidation assistée par impulsion électrique, appelé aussi frittage par arc-plasma.

8. L'implant médical de l'une des revendications précédentes, dans lequel ladite densité relative est d'au moins environ 97% ou d'au moins environ 99%.

9. L'implant médical de l'une des revendications précédentes, dans lequel le rapport entre le métal ou alliage métallique résistant à l'usure et ledit matériau bioactif dans l'une quelconque des couches est de l'ordre de 100:0 à environ 0:100.

10. L'implant médical de l'une des revendications précédentes, dans lequel le rapport entre le métal ou alliage métallique résistant à l'usure et ledit matériau bioactif dans l'une quelconque des couches est de l'ordre de 100:0 à environ 30:70.

11. L'implant médical de l'une des revendications précédentes, dans lequel le rapport entre le métal ou alliage métallique résistant à l'usure et ledit matériau bioactif dans l'une quelconque des couches est de l'ordre de 100:0 à environ 50:50.

12. L'implant médical de l'une des revendications précédentes, dans lequel le nombre de couches est compris entre 4 et 25, ou entre 7 et 20.
